# EUROPÄISCHE PATENTSCHRIFT

(11) **EP 4 413 957 B1**
(45) Veröffentlichungstag und Bekanntmachung des Hinweises auf die Patenterteilung: **01.04.2026**
(21) Anmeldenummer: 24155681.0
(22) Anmeldetag: 05.02.2024
(51) Int. Cl.: A61F 2/44, A61F 2/30, A61F 2/00

(54) **ZERVIKALER CAGE**
CERVICAL CAGE
CAGE CERVICALE

(30) Priorität: 07.02.2023 DE 102023102881
(43) Veröffentlichungstag der Anmeldung: 14.08.2024
(73) Patentinhaber: Maxxspine Innovative GmbH, 65344 Eltville am Rhein (DE)
(72) Erfinder: Ludwig, Volker, 65344 Eltville am Rhein (DE); Röbling, Christian, 65344 Eltville am Rhein (DE)
(74) Vertreter: Tesch, Sabine

(56) Entgegenhaltungen:
- US-A1- 2011 022 173
- US-A1- 2012 232 599
- US-A1- 2021 401 587

## Beschreibung

### Gebiet der Erfindung

Die Erfindung betrifft einen Cage, welcher als Bandscheibenersatz für die Wirbelsäule ausgebildet ist. Insbesondere betrifft die Erfindung einen zervikalen Cage.

### Hintergrund der Erfindung

Zur operativen Wirbelsäulenstabilisierung sind sogenannte Cages bekannt. Diese dienen der Versteifung zweier benachbarter Wirbelkörper und werden anstelle der Bandscheibe zwischen den Wirbeln eingesetzt.

Ein derartiger Cage ist üblicherweise aus Metall, insbesondere Titan, oder aus einem Kunststoff, wie beispielsweise PEEK, ausgebildet. Zur Verbesserung der Fixierung ist es bekannt, Verankerungsschrauben für eine kraniale und kaudale Befestigung quer durch den Cage in die Wirbelkörper zu schrauben.

Weiter kann der Cage innen hohl ausgebildet sein, so dass dieser mit einem Knochenersatzmaterial befüllt werden kann. Bei dem Knochenersatzmaterial handelt es sich zumeist um ein Kalziumphosphatmaterial, in welches natürliches Knochengewebe einwachsen kann.

Zervikale Cages sind aus den Dokumenten US 2012/232599 Al, US 2011/022173 Al und US 2021/401587 Al bekannt.

### Aufgabe der Erfindung

Der Erfindung liegt gegenüber vorstehend genanntem Hintergrund die Aufgabe zugrunde, einen Cage bereitzustellen, welcher flexibel verwendet werden kann. Insbesondere ist es eine Aufgabe der Erfindung, einen verbesserten Cage bereitzustellen, welcher wahlweise mit oder ohne Verankerungsschrauben implantiert werden kann.

### Zusammenfassung der Erfindung

Die Aufgabe der Erfindung wird bereits durch einen Cage mit einer Kappe nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft einen Cage, welcher als Bandscheibenersatz für die Wirbelsäule ausgebildet ist. Insbesondere betrifft die Erfindung einen Cage, welcher für einen zervikalen Bandscheibenersatz ausgebildet ist.

Der Cage umfasst zumindest eine Aufnahme zum Anbringen eines Applikationswerkzeuges.

Die Aufnahme kann insbesondere als Gewinde ausgebildet sein, über das der Cage mit dem Applikationswerkzeug verbunden werden kann. Das Gewinde kann insbesondere an einer Seitenwand, vorzugsweise an einer anterioren Seitenwand des Cages angeordnet sein.

Über die Aufnahme wird der Cage sicher gehalten und kann vom Operateur nach einer Resektion der Bandscheibe zwischen den Wirbeln eingesetzt werden.

Weiter umfasst der Cage zwei Durchführungen für jeweils eine Verankerungsschraube. Die beiden Durchführungen verlaufen insbesondere schräg von unten nach oben bzw. oben nach unten durch den Cage und dienen so einer kaudalen und kranialen Verankerung.

Die Durchführungen können ein Gewinde umfassen. So können die Durchführungen insbesondere im Zusammenwirken mit Verankerungsschrauben verwendet werden, welche ein Kopfgewinde aufweisen und so am Ende des Einschraubvorgangs winkelstabil mit dem Cage verbunden sind.

Gemäß der Erfindung gehört zu dem Cage eine Kappe, mittels welcher die Durchführungen für die Verankerungsschrauben verschließbar sind.

Die Kappen können dann verwendet werden, wenn vom Operateur keine Verankerungsschrauben verwendet werden. So ist der Cage flexibler verwendbar.

Das Verschließen der Durchführungen für die Verankerungsschrauben verbessert des Weiteren das Einbringen von Knochenersatzmaterial in den Cage.

Durch die Kappen wird verhindert, dass Knochenersatzmaterial lateral aus dem Cage austritt.

Als Anschluss zum Injizieren von Knochenersatzmaterial kann beispielsweise die Aufnahme für das Handhabungswerkzeug dienen. Diese kann insbesondere als Gewinde ausgebildet sein, welches sich in das zumindest teilweise hohle Innere des Cage erstreckt.

Durch die Kappen wird ein laterales Austreten des Knochenersatzmaterials blockiert.

Weiter können auch die Durchführungen für die Verankerungsschrauben zum Injizieren von Knochenersatzmaterial verwendet werden. Dabei kann beispielsweise eine Durchführung mittels einer Kappe verschlossen werden und über die andere Durchführung das Knochenersatzmaterial injiziert werden. Über die Durchführungen kann insbesondere gezielt Knochenersatzmaterial oberhalb oder unterhalb des Cage eingebracht werden. Die Kappe ist gemäß der Erfindung als Gewindekappe ausgebildet sein. Vorzugsweise ist die Kappe als Madenschraube ausgebildet. Bei einer derartigen Madenschraube erstreckt sich der Antrieb der Schraube als Sackloch durch das Gewinde.

Die Kappe steht vorzugsweise im eingesetzten Zustand weder kranial noch kaudal aus dem Cage.

Vorzugsweise umfasst die Kappe ein konisches Gewinde. Ein derartiges konisches Gewinde hat den Vorteil, dass die Gewindezüge auf kurzem Weg miteinander in Eingriff kommen. Der Einschraubvorgang erfolgt somit schnell und mit wenig Umdrehungen.

Insbesondere können Cage und Kappe derart ausgebildet sein, dass sich die Kappe mit weniger als zwei Umdrehungen, insbesondere mit 0,3 bis 0,5 Umdrehungen vollständig einschrauben lässt.

Das Gewinde kann insbesondere als einzügiges Gewinde ausgebildet sein.

Die Kappe kann insbesondere kegelstumpfförmig ausgebildet sein. Insbesondere kann der Kegelwinkel α zwischen 50 und 30° liegen.

Die Kappe ist vorzugsweise derart ausgebildet, dass sie sich im eingesetzten Zustand durch die Durchführung erstreckt, aber nicht aus der Durchführung herausschaut.

Es ist insbesondere vorgesehen, dass die Kappe im eingesetzten Zustand eine innere Kante umfasst, welche mit der Unterseite oder Oberseite des Cage fluchtet. Bei der inneren Kante im Sinne der Erfindung handelt es sich insbesondere um den distalen Rand der Kappe, also insbesondere um den distalen Rand der Madenschraube.

Diese Kante liegt im eingesetzten Zustand in etwa auf Höhe der Oberseite bzw. Unterseite des Cage.

Eine äußere Kante dagegen fluchtet vorzugsweise mit der Seitenwand des Cage. Bei der äußeren Kante handelt es sich insbesondere um den proximalen Rand der Kappe.

Bei dieser Ausführungsform erstreckt sich somit die eingesetzte Kappe über eine maximal mögliche Länge. Des Weiteren kann eine Kappe mit verhältnismäßig großem Durchmesser bereitgestellt werden, was es wiederum ermöglicht, Durchführungen für verhältnismäßig große Verriegelungsschrauben bereitzustellen.

Die Durchführungen können sich insbesondere in einem Winkel kranial und/oder kaudal von 30 bis 45° durch den Cage, bezogen auf dessen Mittelebene, erstrecken.

Weiter können sich die Durchführungen lateral in einem Winkel von 5 bis 15° zu einer Mittelachse des Cages erstrecken.

Bei einer Ausführungsform der Erfindung ist der Cage aus gedrucktem Titan oder einer Titanlegierung ausgebildet. Der Cage kann insbesondere als Käfig ausgebildet sein. Dabei kann sich ein Gitter zumindest abschnittweise über die Oberseite oder Unterseite erstrecken. Das Gitter kann von Knochenersatzmaterial durchdrungen werden. Das Gitter kann insbesondere die Form eines Gewebes aufweisen, also Schlaufen umfassen, welche ineinander greifen oder sich umschlingen. Diese Schlaufen sind aber nicht aus Stoff, sondern aus gedrucktem Titan bzw. einer gedruckten Titanlegierung ausgebildet.

Gemäß einem weiteren Aspekt der Offenbarung bezieht sich diese auf einen Cage, welcher als Bandscheibenersatz für die Wirbelsäule ausgebildet ist.

Insbesondere bezieht sich die Offenbarung auf den vorstehend beschriebenen Cage. Der Cage weist zwei Durchführungen auf, die insbesondere zum Durchführen von Verankerungsschrauben dienen können.

Gemäß diesem Aspekt ist an Stelle einer Kappe ein Spike vorgesehen, welcher in zumindest einer Durchführung, vorzugsweise in beiden Durchführungen sitzt.

Der Spike umfasst einen Schaft mit einer Spitze. Der Schaft weist aber kein Gewinde auf. Vielmehr kann der Schaft Widerhaken umfassen, die im Knochengewebe den Spike verankern.

Vorzugsweise umfasst der Spike ein Kopfgewinde, welches in das Gewinde der Durchführung eingeschraubt wird. Das Gewinde kann, wie vorstehend beschrieben, insbesondere konisch ausgebildet sein.

Insbesondere kann der Kopf des Spike wie die vorstehend beschriebene Kappe ausgebildet sein.

Über das Kopfgewinde kann der Spike wesentlich schneller und einfacher als eine Schraube in das angrenzende Knochengewebe geführt und so verankert werden.

Der oder die Spikes können insbesondere vormontiert im Cage sitzen. Es ist insbesondere vorgesehen, dass in dem vormontierten Zustand die Spitze des Spikes nicht aus der Oberseite bzw. Unterseite des Cage herausschaut. Erst mit dem Eindrehen des Kopfgewindes wird die Spitze in den Knochen geführt und verankert so den Cage.

Die Erfindung betrifft des Weiteren ein Set mit einem Cage nebst Kappe und/oder Cage, wie es vorstehend beschrieben wurde. Das Set umfasst des Weiteren eine Verankerungsschraube.

Die Schraube kann insbesondere ein Gewinde mit einem Schaft aufweisen, welches dazu ausgebildet ist, in den angrenzenden Wirbelkörper gedreht zu werden.

Weiter umfasst die Verankerungsschraube vorzugsweise einen Kopf mit einem Kopfgewinde. Das Kopfgewinde kann insbesondere dem Gewinde der Kappe entsprechen.

Die Verankerungsschraube wird anstelle der Kappe verwendet, soweit der Operateur den Cage kaudal und kranial verankern möchte.

Das Set kann des Weiteren ein Applikationswerkzeug mit einem Griff umfassen, welches an der Aufnahme befestigbar ist.

Es kann sich insbesondere um ein Applikationswerkzeug handeln, bei welchem der Griff eine Stange mit einem Gewinde an der Spitze umfasst. An dem Gewinde kann der Cage angebracht werden, um diesen einzubringen.

Weiter kann das Set einen Zementapplikator umfassen, welcher mit einer der Durchführungen und/oder der Aufnahme verbindbar ist, um Knochenersatzmaterial in den Cage zu applizieren.

Es ist insbesondere vorgesehen, dass nach dem Einsetzen des Cages mittels des Applikationswerkzeugs die Aufnahme das einem Kanal, insbesondere einem Schlauch verbunden wird, über den Knochenersatzmaterial in das Innere des Cages appliziert wird.

Bei der Verwendung von Kappen sichern diese, dass das Knochenersatzmaterial nicht an der Seitenwand des Cages austritt.

Weiter kann auch die Durchführung selbst zum Applizieren von Knochenersatzmaterial verwendet werden.

Insbesondere kann die Durchführung seitliche Öffnungen umfassen, so dass Knochenersatzmaterial aus der Durchführung in den Cage eintreten kann. Weiter kann das Knochenersatzmaterial über die Durchführung auch oberhalb und unterhalb unter dem Cage fließen, wo er aufgrund der gitterartigen Struktur der Oberseite und Unterseite in den Cage eindringt.

### Kurzbeschreibung der Zeichnungen

Der Gegenstand der Erfindung soll im Folgenden bezugnehmend auf ein Ausführungsbeispiel anhand der Zeichnungen Fig. 1 bis Fig. 12 näher erläutert werden.
Fig. 1 ist eine perspektivische Ansicht eines Cages ohne eingesetzte Kappen.
Fig. 2 zeigt eine Verankerungsschraube.
Fig. 3 zeigt eine Kappe.
Fig. 4 ist eine perspektivische Ansicht von Cage und Kappe.
Fig. 5 ist ein mittiger Längsschnitt des Cage.
Fig. 6 ist ein Längsschnitt durch eine Durchführung.
Fig. 7 ist ein Längsschnitt, wobei nunmehr die Kappe eingesetzt ist.
Fig. 8 ist eine perspektivische Ansicht eines Applikationswerkzeugs.
Fig. 9 ist eine schematische Darstellung eines Zementapplikators, wie er Bestandteil eines erfindungsgemäßen Sets sein kann.
Fig. 10 illustriert, wie über eine Durchführung des Cage Knochenersatzmaterial eingebracht wird.
Fig. 11 zeigt einen Spike.
Fig. 12 ist eine Schnittansicht des Cages nebst eingesetzten Spikes.

### Detaillierte Beschreibung der Zeichnungen

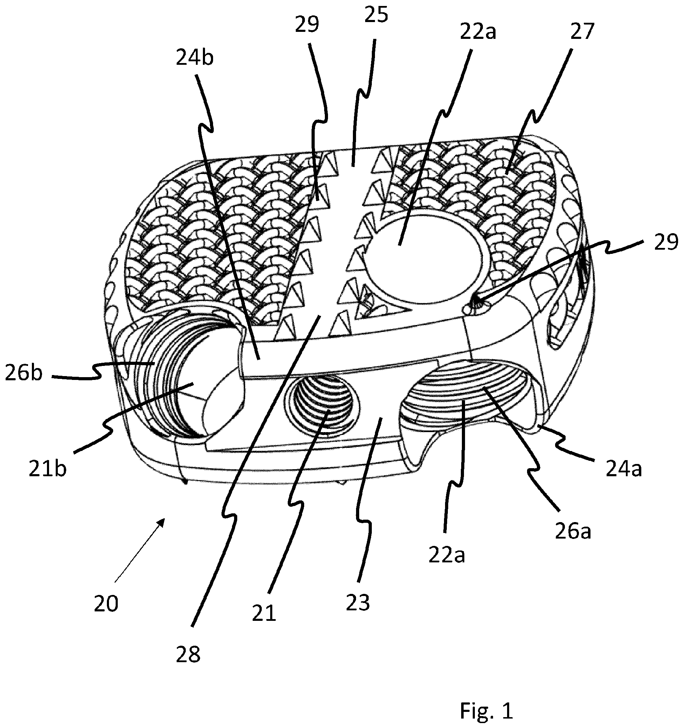

Fig. 1 zeigt in einer perspektivischen Ansicht einen Cage 20.

Der Cage 20 ist als zervikaler Bandscheibenersatz ausgebildet.

Dieser kann insbesondere eine Höhe von 3 bis 10 mm aufweisen (maximale Höhe).

Oberseite 25 und Unterseite verlaufen gemäß einer bevorzugten Ausführungsform nicht parallel, sondern schräg zueinander. Diese bilden einen sogenannten Lordosewinkel. Der Lordosewinkel kann beispielsweise zwischen 2 und 8° liegen.

Der Cage 20 ist aus einer gedruckten Titanlegierung ausgebildet (z.B. mittels SLM hergestellt).

Oberseite 25 und Unterseite des Cages 20 sind zumindest abschnittsweise mit einer Gitterstruktur 27 versehen. So sind Oberseite 25 und Unterseite für Knochenersatzmaterial durchlässig. Dazwischen befindet sich ein Hohlraum.

Weiter erstreckt sich ein Steg 28 in etwa mittig über den Cage 20. Es erstreckt sich sowohl auf der Oberseite 25 als auch auf der Unterseite ein Steg 28, welcher der Verstärkung dient.

Der Cage kann an seiner Oberseite 25 und/oder Unterseite des Weiteren Spitzen 29 umfassen, welche sich in die Knochensubstanz eindrücken und so die Fixierung des Cages 20 verbessern.

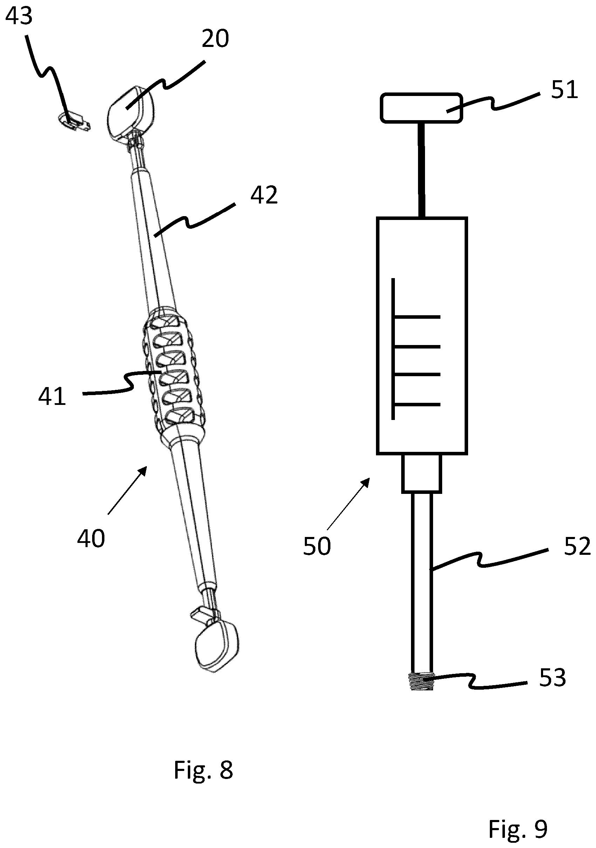

Als Aufnahme 21 für ein Applikationswerkzeug umfasst die Seitenwand 23 des Cage 20 ein Gewinde. So kann ein Applikationswerkzeug (siehe Fig. 8) in den Cage 20 geschraubt werden und der Cage kann platziert werden.

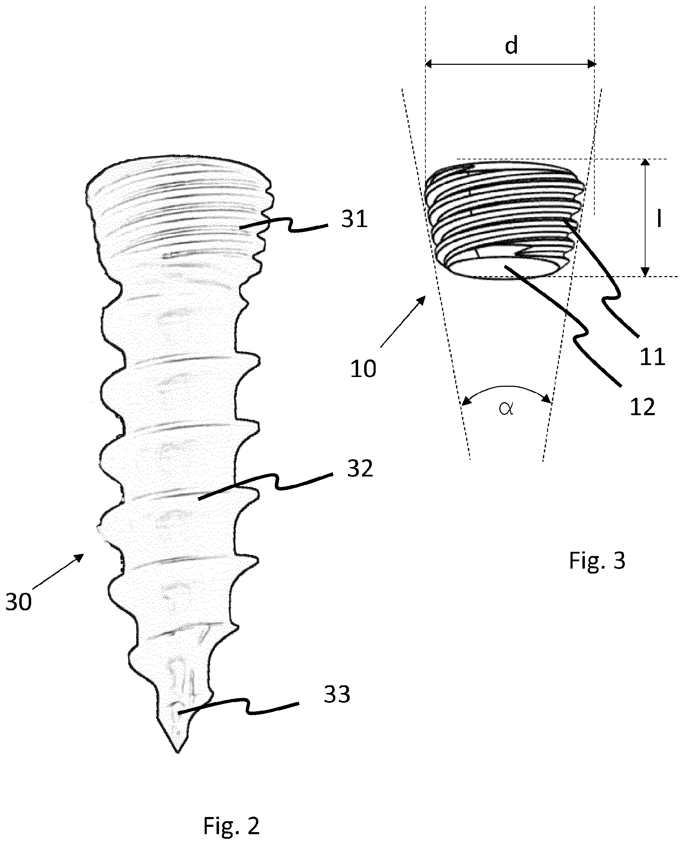

Zur kranialen und kaudalen Verankerung mittels einer Verankerungsschraube (30 in Fig. 2) umfasst der Cage 20 Durchführungen 22a, 22b, welche von der Seitenwand ausgehend schräg durch den Cage 20 verlaufen.

Die Durchführungen 22a, 22b umfassen jeweils ein Gewinde 26a, 26b, in welchem sich entweder der Kopf der Verriegelungsschraube verankert oder in welches eine Kappe (siehe Fig. 3) eingesetzt wird.

Die Durchführung 22a führt zur Oberseite 25 und die Durchführung 22b zur Unterseite.

Fig. 2 zeigt eine Verriegelungsschraube 30.

Die Verriegelungsschraube 30 umfasst einen Kopf mit einem Kopfgewinde 31.

Ausgehend vom Kopfgewinde 31 erstreckt sich ein Schaft, welcher ein Gewinde 32 umfasst, das in die Knochensubstanz gedreht werden kann. Die Verriegelungsschraube 30 kann eine selbstschneidende Spitze 33 umfassen.

Statt einer Verriegelungsschraube 30, wie in Fig. 2 dargestellt, kann die in Fig. 3 dargestellte Kappe 10 verwendet werden.

Die Kappe ist kegelstumpfförmig ausgebildet und umfasst ein konisches Außengewinde 11. Die Unterseite 12 der Kappe 10 ist verschlossen.

Das Außengewinde 11 entspricht vorzugsweise dem Kopfgewinde der Verriegelungsschraube (31 in Fig. 2).

Die Kappe kann einen Durchmesser d (maximaler Durchmesser) von 3 bis 8 mm, vorzugsweise 4 bis 6 mm aufweisen. Die Länge 1 der Kappe 10 kann 3 bis 10 mm betragen.

Der Kegelwinkel α beträgt vorzugsweise 15 bis 30°.

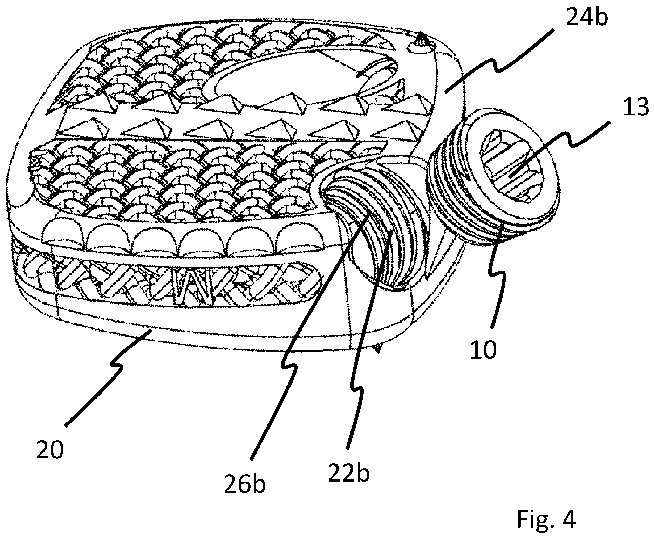

Fig. 4 ist eine perspektivische Ansicht des Cages, in den nunmehr eine Kappe 10 eingesetzt werden soll.

Die Kappe 10 wird in das Gewinde 26b der Durchführung 22b geschraubt.

Die Kappe 10 ist als Madenschraube ausgebildet und hat mithin einen Antrieb 13, welcher sich als Sackloch durch das Gewinde der Kappe 10 erstreckt. Der Antrieb 13 kann beispielsweise als Innenvielzahn ausgebildet sein.

Die Kappe 13 ist vorzugsweise aus einem Vollmaterial aus Metall, insbesondere aus Titan oder einer Titanlegierung, ausgebildet.

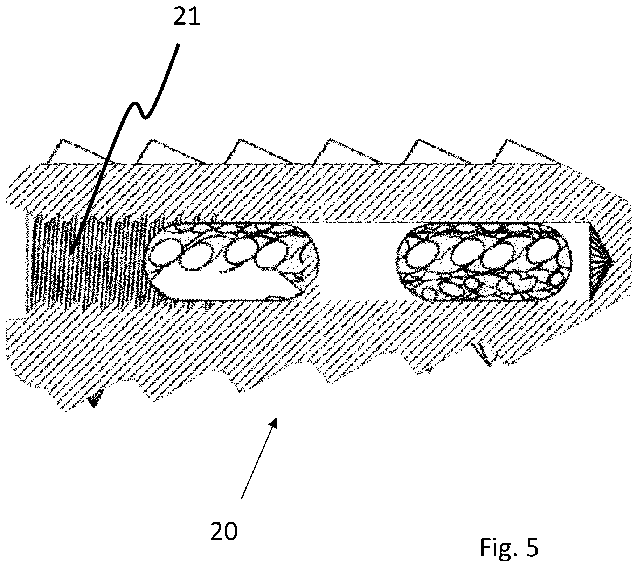

Fig. 5 ist ein Längsschnitt mittig durch den Cage 10.

Zu erkennen ist, dass die Aufnahme 21 in den Cage hineinführt. Über die Aufnahme 21 kann so Knochenersatzmaterial in den Hohlraum zwischen Oberseite und Unterseite injiziert werden.

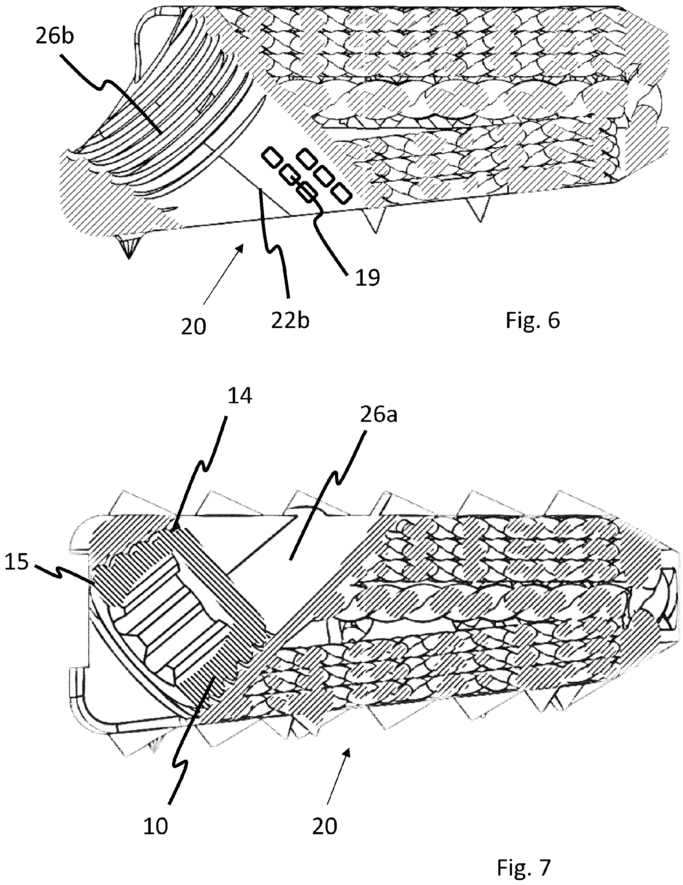

Fig. 6 ist ein Längsschnitt des Cages im Bereich einer Durchführung 22b.

Die Durchführung 22b mit dem Gewinde 26b kann, wie es bei einer weiteren Ausführungsform vorgesehen ist, seitliche Öffnungen 19 umfassen, über die Knochenersatzmaterial in den Cage 20 hineintreten kann. Die Öffnungen 19 sind in dieser Ansicht schematisch dargestellt.

Das Gewinde 26b ist konisch ausgebildet. Aufgrund der Schrägstellung der Durchführung 22b kann sich das Gewinde nur über einen Teilbereich der Durchführung 22b erstrecken. Vorzugsweise erstreckt sich das Gewinde 26b von der Oberseite des Cage bis zu dessen Unterseite.

Fig. 7 zeigt, wie nunmehr eine Kappe 10 in eine Durchführung 26a eingeschraubt ist.

Die Kappe 10 umfasst eine äußere, proximale Kante 15, welche in etwa mit der Seitenwand des Cages 20 fluchtet.

Die innere, distale Kante 14 fluchtet in etwa mit der Ober- oder Unterseite des Cages 20.

Fig. 8 ist eine perspektivische Ansicht eines Applikationswerkzeugs 40. Das Applikationswerkzeug 40 umfasst einen Griff 41, der eine Stange 42 umfasst, an deren Ende ein Gewinde angeordnet ist, auf das der Cage 20 geschraubt werden kann.

In diesem Ausführungsbeispiel umfasst das Applikationswerkzeug 40 beidseitig des Griffes 41 jeweils eine Stange 42, an der ein Cage 20 angebracht werden kann.

Das Applikationswerkzeug 40 kann des Weiteren einen Abstandshalter 43 umfassen.

Der Abstandshalter 43 kann optional in die Stange 42 eingesetzt, insbesondere verrastet werden. Der Abstandshalter 43 verhindert, dass der Cage 20 zu tief zwischen die Wirbel eingeführt wird, indem dieser am angrenzenden Wirbelkörper zur Anlage kommt.

Fig. 9 zeigt in einer schematischen Darstellung einen Zementapplikator 50.

Der Zementapplikator 50 umfasst einen Griff 51, über den, beispielsweise durch Drücken oder Drehen, Knochenersatzmaterial ausgepresst werden kann.

Das Knochenersatzmaterial tritt über den Kanal 52, welcher beispielsweise als Schlauch ausgebildet ist, aus dem Zementapplikator 50 heraus.

Der Kanal 52 umfasst an seinem distalen Ende ein Gewinde 53.

Dieses Gewinde 53 kann beispielsweise in die Aufnahme des Cages eingedreht werden, nachdem das Applikationswerkezeug herausgedreht wurde.

Gemäß einer weiteren Ausführungsform kann das Gewinde 53 dem Außengewinde der Kappe entsprechen, wobei so die Durchführungen zum Applizieren von Knochenersatzmaterial verwendet werden können.

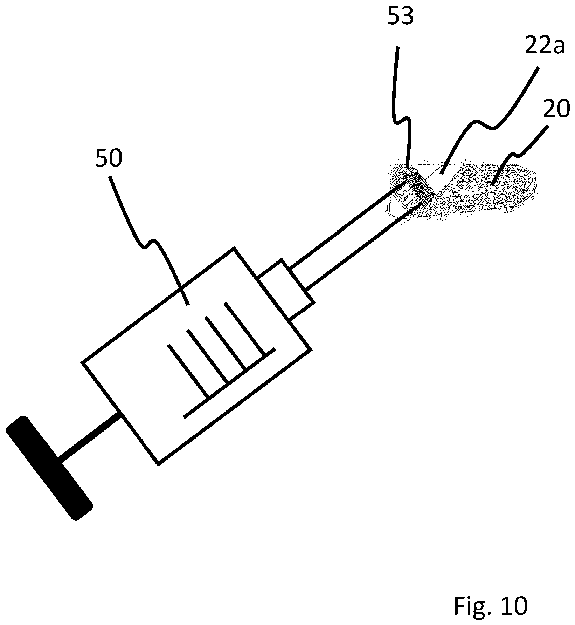

Fig. 10 illustriert, wie mittels des Zementapplikators 50 Knochenersatzmaterial durch eine Durchführung 22a des Cage 20 eingebracht wird.

Der Zementapplikator 50 ist mit dem Gewinde 53 in die Durchführung 22a geschraubt.

Auf diese Weise kann Knochenersatzmaterial eingebracht werden, welches sich vorwiegend zunächst oberhalb oder unterhalb des angrenzenden Wirbels verteilt. So können insbesondere Höhenunterschiede zwischen Wirbel und Cage egalisiert werden. Es kommt zu einer gleichmäßigeren Druckverteilung.

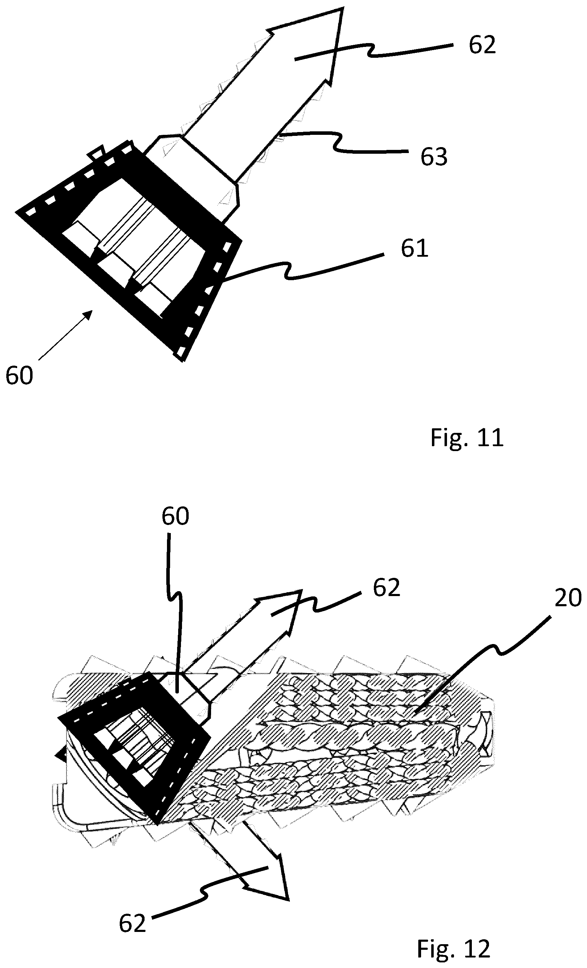

Fig. 11 zeigt schematisch einen Spike 16, welcher gemäß einem weiteren Aspekt der Erfindung anstelle der Kappe vorgesehen ist.

Der Spike 60 umfasst ein Kopfgewinde 61, welches insbesondere entsprechend vorstehend beschriebener Kappe ausgebildet sein kann.

An das Kopfende grenzt bei dem Spike 60 allerdings eine Spitze 62 an, welche am Ende des Schaftes ist.

Der Schaft kann mit Widerhaken 63 versehen sein.

Wie in Fig. 12 dargestellt, können die Spikes 60 mit dem Kopfgewinde eingedreht werden und verankern sich im Cage 20.

Die Spitze 62 des jeweiligen Spikes 60 drückt sich so ins Knochengewebe und verankert den Cage 20.

Gegenüber einer Verankerungsschraube geht die Verankerung schneller vonstatten. Auch ist denkbar, die Spikes 60 bereits im Cage 20 vorzumontieren, dass die Spitze 62 noch nicht herausschaut, wenn der Cage 20 eingesetzt wird.

Durch die Erfindung konnte ein flexibel verwendbarer Cage bereitgestellt werden.

### Bezugszeichenliste

- 10: Kappe
- 11: Außengewinde
- 12: Unterseite
- 13: Antrieb
- 14: innere Kante
- 15: äußere Kante
- 19: Öffnung
- 20: Cage
- 21: Aufnahme
- 22a, 22b: Durchführung
- 23: Seitenwand
- 24a, 24b: Ecke
- 25: Oberseite
- 26a, 26b: Gewinde
- 27: Gitterstruktur
- 28: Steg
- 29: Spitze
- 30: Verankerungsschraube
- 31: Kopfgewinde
- 32: Gewinde
- 33: selbstschneidende Spitze
- 40: Applikationswerkzeug
- 41: Griff
- 42: Stange
- 43: Abstandshalter
- 50: Zementapplikator
- 51: Griff
- 52: Kanal
- 53: Gewinde
- 60: Spike
- 61: Kopfgewinde
- 62: Spitze
- 63: Widerhaken

## Patentansprüche

1. Cage (20), welcher als Bandscheibenersatz für die Wirbelsäule ausgebildet ist, insbesondere als zervikaler Bandscheibenersatz,
wobei der Cage (20) zumindest eine Aufnahme (21) zum Anbringen eines Applikationswerkzeugs (40) umfasst,
und wobei der Cage (20) zumindest zwei Durchführungen (22a, 22b) für jeweils eine Verankerungsschraube (30) umfasst,
wobei eine Kappe (10) vorgesehen ist, mittels welcher die Durchführungen (22a, 22b) für die Verankerungsschrauben (30) verschließbar sind,
**dadurch gekennzeichnet, dass** die Kappe (10) als Gewindekappe ausgebildet ist, welche in ein Gewinde (26a, 26b) der Durchführung (22a, 22b) geschraubt ist.

2. Cage (20) nach dem vorstehenden Anspruch, **dadurch gekennzeichnet, dass** die Kappe (10) ein konisches Gewinde (32) umfasst.

3. Cage (20) nach einem der vorstehenden Ansprüche, **dadurch gekennzeichnet, dass** die Kappe (10) kegelstumpfförmig ausgebildet ist, insbesondere mit einem Kegelwinkel α von 15 bis 30°.

4. Cage (20) nach einem der vorstehenden Ansprüche, **dadurch gekennzeichnet, dass** bei einer eingesetzten Kappe (10) diese eine innere Kante (14) umfasst, welche mit der Unterseite (12) oder Oberseite (25) des Cage (20) fluchtet, wobei eine äußere Kante (15) mit der Seitenwand des Cage (20) fluchtet.

5. Cage (20) nach einem der vorstehenden Ansprüche, **dadurch gekennzeichnet, dass** die Durchführungen (22a, 22b) sich schräg durch den Cage (20) erstrecken.

6. Cage nach dem vorstehenden Anspruch, **dadurch gekennzeichnet, dass** die Durchführungen (22a, 22b) sich schräg in einem Winkel kranial und/oder kaudal von 30 bis 45° durch den Cage (20) erstrecken.

7. Cage (20) nach einem der vorstehenden Ansprüche,
**dadurch gekennzeichnet, dass** die Durchführungen (22a, 22b) lateral in einem Winkel von 5 bis 15° zur Mittelachse angeordnet sind.

8. Cage (20) nach einem der vorstehenden Ansprüche, **dadurch gekennzeichnet, dass** der Cage (20) als Käfig aus gedrucktem Titan oder einer Titanlegierung ausgebildet ist.

9. Set mit einem Cage (20) nebst Kappe (10) nach einem der vorstehenden Ansprüche, weiter umfassend zumindest eine Verankerungsschraube (30).

10. Set nach dem vorstehenden Anspruch, weiter umfassend ein Applikationswerkzeug mit einem Griff, welches an der Aufnahme befestigbar ist.

11. Set nach einem der vorstehenden Ansprüche, weiter umfassend einen Zementapplikator (50), der mit einer der Durchführungen (22a, 22b) und/oder der Aufnahme verbindbar ist, um Knochenersatzmaterial in den Cage (20) zu applizieren.

## Claims

1. A cage (20), which is designed as an intervertebral disc replacement for the spine, in particular as a cervical intervertebral disc replacement,
wherein the cage (20) comprises at least one receptacle (21) for securing an application tool (40), and wherein the cage (20) comprises at least two passages (22a, 22b) each one for a respective anchoring screw (30),
wherein a cap (10) is provided by means of which the passages (22a, 22b) for the anchoring screws (30) can be closed,
**characterised in that** the cap (10) is in the form of a threaded cap which is screwed into a thread (26a, 26b) of the passage (22a, 22b).

2. The cage (20) according to the preceding claim, **characterised in that** the cap (10) comprises a conical thread (32).

3. The cage (20) according to any one of the preceding claims, **characterised in that** the cap (10) has a frustoconical shape, in particular with a cone angle α of 15° to 30°.

4. The cage (20) according to any one of the preceding claims, **characterised in that** the cap (10), in its inserted state, has an inner edge (14) which is flush with the lower surface (12) or upper surface (25) of the cage (20), while an outer edge (15) is flush with the side wall of the cage (20).

5. The cage (20) according to any one of the preceding claims, **characterised in that** the passages (22a, 22b) extend obliquely through the cage (20).

6. The cage according to the preceding claim, **characterised in that** the passages (22a, 22b) extend obliquely through the cage (20) at an angle of 30° to 45° cranially and/or caudally.

7. The cage (20) according to any one of the preceding claims, **characterised in that** the passages (22a, 22b) are arranged laterally at an angle of 5 to 15° relative to the central axis.

8. The cage (20) according to any one of the preceding claims, **characterised in that** the cage (20) is in the form of a cage made of printed titanium or a titanium alloy.

9. A kit comprising a cage (20) and a cap (10) according to any one of the preceding claims, further comprising at least one anchoring screw (30).

10. The kit according to the preceding claim, further comprising an application tool with a handle, which tool can be secured in the receptacle.

11. The kit according to any one of the preceding claims, further comprising a cement applicator (50), which can be connected to one of the passages (22a, 22b) and/or to the receptacle in order to apply bone substitute material into the cage (20).

## Revendications

1. Cage (20), conçu comme un remplacement de disque intervertébral pour la colonne vertébrale, en particulier comme un remplacement de disque intervertébral cervical,
le cage (20) comprenant au moins un logement (21) destiné à la fixation d'un instrument d'application (40), et le cage (20) comprenant au moins deux passages (22a, 22b) chacun destinés à une vis d'ancrage (30),
une coiffe (10) étant prévue, au moyen de laquelle les passages (22a, 22b) pour les vis d'ancrage (30) peuvent être fermés,
**caractérisé en ce que** la coiffe (10) est réalisée sous la forme d'une coiffe filetée qui est vissée dans un filetage (26a, 26b) du passage (22a, 22b).

2. Cage (20) selon la revendication précédente, **caractérisé en ce que** la coiffe (10) comprend un filetage conique (32).

3. Cage (20) selon l'une quelconque des revendications précédentes, **caractérisé en ce que** la coiffe (10) est réalisée sous la forme d'un tronc de cône, en particulier avec un angle de cône α de 15° à 30°.

4. Cage (20) selon l'une quelconque des revendications précédentes, **caractérisé en ce que**, lorsque la coiffe (10) est mise en place, celle-ci comprend un bord intérieur (14) affleurant avec la face inférieure (12) ou la face supérieure (25) du cage (20), un bord extérieur (15) affleurant avec la paroi latérale du cage (20).

5. Cage (20) selon l'une quelconque des revendications précédentes, **caractérisé en ce que** les passages (22a, 22b) s'étendent de manière oblique à travers le cage (20).

6. Cage selon la revendication précédente, **caractérisé en ce que** les passages (22a, 22b) s'étendent de manière oblique à travers le cage (20) selon un angle crânial et/ou caudal de 30° à 45°.

7. Cage (20) selon l'une quelconque des revendications précédentes, **caractérisé en ce que** les passages (22a, 22b) sont disposés latéralement selon un angle de 5 à 15° par rapport à l'axe central.

8. Cage (20) selon l'une quelconque des revendications précédentes, **caractérisé en ce que** le cage (20) est réalisé sous la forme d'un cage en titane imprimé ou en alliage de titane.

9. Ensemble comprenant un cage (20) et une coiffe (10) selon l'une quelconque des revendications précédentes, comprenant en outre au moins une vis d'ancrage (30).

10. Ensemble selon la revendication précédente, comprenant en outre un instrument d'application avec une poignée, l'instrument pouvant être fixé au logement.

11. Ensemble selon l'une quelconque des revendications précédentes, comprenant en outre un applicateur de ciment (50), qui est raccordable à l'un des passages (22a, 22b) et/ou au logement afin d'appliquer un matériau de substitution osseuse dans le cage (20).
